# EUROPÄISCHE PATENTSCHRIFT

(11) **EP 0 624 378 B1**
(45) Veröffentlichungstag und Bekanntmachung des Hinweises auf die Patenterteilung: **03.06.1998**
(21) Anmeldenummer: 94107049.2
(22) Anmeldetag: 05.05.1994
(51) Int. Cl.: A61L 2/26, A61B 19/02

(54) **Sterilisations-Doppelsack**
Double bag for sterilization
Sac double pour la stérilisation

(30) Priorität: 12.05.1993 DE 9307217 U
(43) Veröffentlichungstag der Anmeldung: 17.11.1994
(73) Patentinhaber: ODENWALD CHEMIE GmbH, 69246 Schönau/Heidelberg (DE)
(72) Erfinder: Schindler, Wilhelm, D-69190 Walldorf (DE); Riemann, Roland, D-69250 Schönau (DE); Weiss, Edgar, D-69239 Neckarsteinach (DE)
(74) Vertreter: Müller, Hans-Jürgen, Dipl.-Ing.

(56) Entgegenhaltungen:
- DE-A- 3 717 916
- DE-U- 9 212 865
- DE-U- 9 217 097
- US-A- 5 078 508

## Beschreibung

Die Erfindung bezieht sich auf einen doppelwandigen Sack für infektiösen Müll oder dergleichen der im Oberbegriff des Anspruchs 1 genannten Gattung.

Ein derartiger Abfallsack ist bereits bekannt (DE-GM 92 12 865). Dabei ist der untere flüssigkeitsdichte Sackteil oben mit einem gasdurchlässigen Teil in Form eines Filtermaterials aus Kunststoff längs einer thermischen Siegelnaht verbunden. Der untere flüssigkeitsdichte Teil ist doppelwandig und besteht aus einer dünnen Trägerschicht und einer dickeren Siegelungsschicht. Während die Trägerschicht aus einem hochschmelzenden Kunststoff gebildet ist, der erst weit oberhalb 134°C schmilzt, erschmilzt die dickere Siegelungsschicht bei geringeren Temperaturen. Derartige Müllsäcke dienen zum Sammeln infektiösen Mülls, wie er beispielsweise in Krankenhäusern anfällt. Solcher Müll wird in dem Sack gesammelt, worauf der Müllsack verschlossen wird. Bei etwa 134°C findet eine Dampfsterilisation statt, bei der der Dampf durch das gasdurchlässige Filtermaterial hindurchtritt und die Keime des Mülls innerhalb des Müllsacks abtötet. Nach dem Sterilisieren kann der Müllsack mit dessen Inhalt der weiteren Entsorgung zugeführt werden, ohne daß dort infektiöse Keime die Entsorgung beeinträchtigen. Die Filterschicht bildet jedoch keine genügende Sicherheit gegen das Austreten von Keimen aus dem verschlossenen Müllsack.

Darüber hinaus ist es bekannt (US-A-4 644 586), den Sack in seiner Längsrichtung aus unterschiedlichen Materialien zusammenzusetzen, um einerseits einen gasdurchlässigen und anderereits unmittelbar daran anschließend einen gasundurchlässigen Bereich auszubilden. Die Handhabung des Mülls innerhalb des Sackes muß jedoch durch Verlagern besonders sorgfältig erfolgen, da sonst die Aufgabe nicht erfüllt werden kann. Außerdem ist es bekannt (US-A-4 828 892), den Sack als Laminat von zwei oder drei Schichten aus unterschiedlichen Polyolefinen zusammenzusetzen, u.z. außen aus insb. HD-PE und innen aus MD- oder LD-PE, das weniger temperaturbeständig als die Außenschicht ist; alle Schichten sind gasdurchlässig und die Keimaustrittsgefahr aus dem Sack ist groß.

Schließlich ist es bekannt (DE-A-3 717 916), einen Doppelsack aus einem teilweise gasdurchlässigen Außensack und einem teilweise gasdurchlässigen Innensack mit besonderer Anordnung gasdurchlässiger und gasundurchlässiger Bereiche zusammenzusetzen, um eine in Sacklängsrichtung erfolgende Gaszirkulation bei vom Innensack beabstandeten Außensack zu erreichen. Auch hier ist die Gefahr von Keimaustritt nicht gebannt.

Der Erfindung liegt die Aufgabe zugrunde, einen Doppelsack der eingangs genannten Gattung noch weiter zu verbessern, so daß dieser im verschlossenen Zustand größte Sicherheit gegen das Austreten infektiöser Keime aus dem verschlossenen Müllsack garantiert, dennoch aber eine einfache Handhabung und spätere problemlose Entsorgung nach dem Sterilisieren bzw. Desinfizieren bietet.

Die Erfindung ist im Anspruch 1 gekennzeichnet und in Unteransprüchen sind weitere Ausbildungen derselben beansprucht. Darüber hinaus ergeben sich aus der folgenden Figurenbeschreibung und Zeichnung weitere besonders bevorzugte Ausführungsbeispiele.

Gemäß der Erfindung stellt der Abfallsack einen "Doppelsack" bestehend aus einem Außensack und einem darin angeordneten Innensack dar. Der Innensack ist flüssigkeitsdicht und keimundurchlässig, während der Außensack Durchbrechungen, insb. Löcher und/oder Schlitze, aufweist, durch welche Gase und insb. zum Sterilisieren dienender Dampf frei, ohne erhebliche zeitliche Verzögerungen, hindurchtreten kann. Der flüssigkeitsdichte Innensack besteht aus einem Material, das wesentlich weniger temperaturbeständig als das Material des gasdurchlässigen Außensackes ist. Dabei braucht der Außensack nicht aus mehreren Schichten zu bestehen; vielmehr genügt eine einzige Kunststoffolie, die dem heißen Sterilisierungsdampf standhält, während der Innensack beim Hindurchtreten des Heißdampfes durch die Durchbrechungen im stabileren Außensack schmilzt bzw. seine Festigkeit jedenfalls soweit verliert, daß er bei einer auf ihn einwirkenden Temperatur von insb. etwa 60-70°C so zerstörbar ist, daß der sterilisierende Heißdampf mit Temperaturen erheblich oberhalb dieser Temperatur den Innensack unter Umständen mit Hilfe von Vakuum zerstört. Dadurch erhält der Heißdampf Zutritt zum Innenraum des Innensackes und kann die dort befindliche Keime abtöten bzw. sterilisieren.

Das Erweichen des Innensackes bis hin zur Selbstzerstörung aufgrund seiner geringen Temperaturbeständigkeit kann auch auf andere Weise als durch den heißen Sterilisierungsdampf erfolgen, der erst zum eigentlichen Sterilisieren, d.h. nach dem mindestens teilweise Zerstören des Innensackes, angewendet wird und sogar durch andere Sterilisierungsmittel ersetzt werden könnte.

Ein weiterer Vorteil der Erfindung besteht darin, daß eine Beschädigung des Außensackes zu keiner Beeinträchtigung der Funktion des Müllsackes führt. Da der Außensack bereits Löcher aufweist, spielt es keine Rolle, ob zusätzliche Risse im Außensack entstehen, sofern dieser für den Innensack noch einen genügenden mechanischen Schutz bietet, um das Aufreißen des Innensacks vor dem Sterilisieren zu verhindern. Insofern bietet der erfindungsgemäße Doppelsack ein Höchstmaß an Sicherheit gegen leichte mechanische Beschädigung im Rahmen von z.B. Lade- und Transportvorgängen, bevor die Sterilisationsstationen erreicht werden. Neben der raumsparenden einfachen Handhabung und der kostengünstigen Herstellung des Doppelsacks wird aufgrund der Addition der Wanddicken der beiden Einzelsäcke eine Gesamtwandstärke von z.B. 0,15 mm erreicht, so daß die Dickenanforderung bezüglich arbeitsschutzrechtlicher Durchführungsanweisungen im Hinblick auf ein "sicheres Umschließen" des infektösen Abfalls erfüllt wird.

Sofern der Außensack aus durchsichtigem transparentem Material besteht und der Innensack insbesondere gefärbt ist, läßt sich optisch sehr leicht der Zustand des Innensackes, der im übrigen vom Außensack vollständig umgeben ist, überprüfen.

Beide Einzelsäcke werden bevorzugt aus chlorfreien Kunststoffen hergestellt, so daß die Entsorgung insbesondere bei thermischer Verwertung umweltfreundlich möglich ist. Im Gegensatz zu laminierten Verbundfolien, die kaum sinnvoll recycled werden können, ist es möglich, Innen- und Außensack voneinander zu trennen, so daß sortenreine Kunststoffe einem sinnvollen Recycling zugeführt werden können. Dieser Aspekt ist nicht nur bei nicht mehr benötigten Sterilisations-Doppelsäcken, sondern auch im Hinblick auf die anfallenden Produktionsabfälle zum Erreichen einer positiven Ökobilanz von Bedeutung.

Ein Ausführungsbeispiel der Erfindung wird nun anhand der Zeichnung erläutert. Dabei zeigen:
- Figur 1: eine schematische Aufsicht auf den erfindungsgemäßen Doppelsack in dessen ungefülltem Zustand, in dem die beiden Säcke im flachen Zustand zu dem Doppelsack vereint sind;
- Figur 2: den Doppelsack im gefüllten und verschlossenen Zustand und
- Figur 3: einen vergrößerten Teilschnitt des Doppelsacks zur Darstellung des Aufbaus und der Anordnung des Innensacks innerhalb des Außensackes.

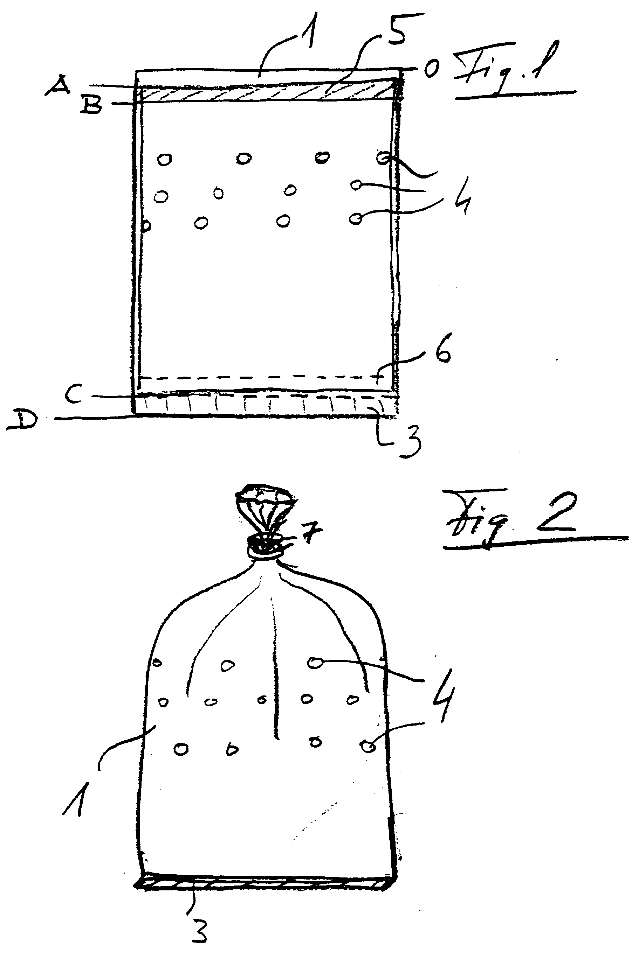

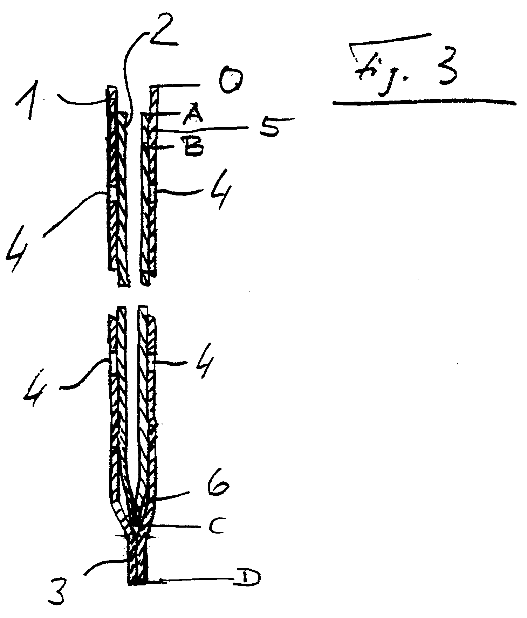

Gemäß Figuren 1 und 3 erstreckt sich ein durchsichtiger Außensack 1 im flachen Zustand zwischen der oberen Begrenzungslinie O und der unteren Begrenzungslinie D. Der Außensack 1 ist unten längs der Verschlußnaht 3 zwischen den Begrenzungslinien C und D durch thermisches Versiegeln verschlossen, am oberen Rand dagegen offen, wie dies deutlicher in Figur 3 gezeigt ist.

Innerhalb des mit Durchbrechungen, wie Schlitzen oder wie hier mit Löchern 4 versehenen Außensackes 1 ist der insbesondere gefärbte, beispielsweise gelbe Innensack 2 angeordnet. Die Löcher 4 sind bevorzugt im mittleren Drittel der Sackhöhe, also nicht im unteren Drittel und möglichst nicht im oberen Drittel angeordnet. Der Innensack 2 ist im unteren Teil mit einer Verschlußnaht 6 verschlossen, die sich auf der Verschlußnaht 3 des Außensackes 1 abstützen kann. Während der Innensack 2 lose innerhalb des Außensacks 1 angeordnet und dort auch von der Innenwand des Außensackes abhebbar ist, empfiehlt es sich bei der bevorzugten Ausbildung der Erfindung nach der Zeichnung, den Innensack 2 an dessen oberen Rand längs einer Verbindungsnaht 5 zwischen den Begrenzungslinien A und B -etwas einwärts des oberen Begrenzungsrandes O des Außensacks 1 mit dem Außensack 1 -zu verbinden. Außensack 1 und Innensack 2 bilden dann trotz der getrennten Anordnung eine oben zusammenhängende Einheit. Die beiden Säcke 1, 2 sind längs dieses Verbindungsrandes 5 (gemäß Figur 3 an beiden Seitenwänden des Außensacks 1 bzw. Innensacks 2) miteinander verbunden, sonst aber nur lose aneinander anliegend.

Gemäß Figur 2 ist der Doppelsack mit infektiösem Müll gefüllt und am oberen Rand mittels eines Verschlusses 7 gasundurchlässig verschlossen, so daß keine sich innerhalb des Innensacks 2 im Müll befindlichen Keime nach außen austreten können. Der verschlossene Doppelsack wird in diesem Zustand, in dem sich der Innensack 2 auf der unteren Verschlußnaht 3 des Außensacks 1 abstützt, zur Desinfektionsstation befördert. Dort wird der gesamte Doppelsack einer erhöhten Temperatur von beispielsweise 100-135 °C ausgesetzt mit der Folge, daß zwar der Außensack 1 dieser Temperatur hinsichtlich seiner Stabilität standhält, der Innensack durch Erweichen aber seine mechanische Festigkeit jedenfalls soweit verliert, daß er an einigen Stellen unmittelbar und/oder nach Anlegen eines äußeren Unterdrucks bzw. Vakuums aufreißt. Anschließend findet die Sterilisation des im aufgerissenen Innensack 2 befindlichen Mülls statt, was vorzugsweise durch Einleiten von heißem Dampf geschieht, der gleichzeitig das Mittel zum Erweichen des Innensacks 2 darstellen kann.

Der Innensack 2 besteht bevorzugt aus einem thermoplastischen Kunststoff aus speziellem Polyethylen PE, während der gelochte Außensack aus einlagigem thermoplastischen Kunststoff, insb. aus Polypropylen PP, aber z.B. auch aus Polyamid PA, gebildet ist.

## Patentansprüche

1. Sterilisations-Doppelsack für infektiösen Müll oder dergleichen mit einem flüssigkeitsdichten und im wesentlichen keimundurchlässigen Teil aus Kunststoff und mit einem gasdurchlässigen Teil, welche Teile insbesondere längs einer Verbindungsnaht miteinander verbunden sind,
**dadurch gekennzeichnet**,
daß beide Teile als getrennte Säcke (1,2) ausgebildet und derart vereint sind, daß der flüssigkeitsdichte Teil als Innensack (2) innerhalb des gasdurchlässigen Außensacks (1) angeordnet und mit der Maßgabe wesentlich weniger temperaturbeständig als der gasdurchlässige Außensack (1) ausgebildet ist, daß der Innensack (2) seine mechanische Festigkeit während der Sterilisation soweit verliert, daß er aufreißt.

2. Doppelsack nach Anspruch 1,
**dadurch gekennzeichnet**,
daß der Außensack (1) Durchbrechungen, insb. Löcher (4) oder Schlitze, aufweist.

3. Doppelsack nach Anspruch 1 oder 2,
**dadurch gekennzeichnet**,
daß der Außensack (1) transparent ist.

4. Doppelsack nach einem der vorhergehenden Ansprüche,
**dadurch gekennzeichnet**,
daß der Innensack (2) farbig ist.

5. Doppelsack nach einem der vorhergehenden Ansprüche,
**dadurch gekennzeichnet**,
daß der Innensack (2) am oberen Rand längs einer Verbindungsnaht (5) mit dem Außensack (1) thermoversiegelt, vernäht oder verklebt ist.

6. Doppelsack nach einem der verhergehenden Ansprüche,
**dadurch gekennzeichnet,**
daß der Außensack (1) länger als der Innensack (2) ausgebildet ist und den letztgenannten am unteren und am oberen Rand überragt.

7. Doppelsack nach Anspruch 6,
**dadurch gekennzeichnet**,
daß sich der Innensack (2) auf einer unteren Verschlußnaht (3) des Außensacks (1) abstützt.

8. Doppelsack nach einem der vorhergehenden Ansprüche,
**dadurch gekennzeichnet,**
daß das Material des Innensacks (2) bei Temperaturen zwischen 60 und 135 °C erweicht oder schmilzt.

9. Doppelsack nach Anspruch 8,
**dadurch gekennzeichnet,**
daß der Innensack (2) aus einem Polyethylen besteht, das bereits bei etwa 60 - 70 °C erweicht oder schmilzt.

10. Doppelsack nach einem der vorhergehenden Ansprüche,
**dadurch gekennzeichnet,**
daß sowohl der Außensack (1) als auch der Innensack (2) aus chlorfreiem Kunststoff bestehen.

## Claims

1. Dual sterilisation bag for infectious waste or the like, comprising a liquid-tight section of synthetic material, which is essentially impermeable to germs, and a gas-permeable section, which sections are connected to each other particularly along a joining seam,
**characterised in**
that said two sections are configured as separate bags (1, 2) and are united in a way that said liquid-tight section is disposed as interior bag (2) in said said gas-permeable exterior bag (1) and is essentially less temperature-resistant than said gas-permeable exterior bag (1) on the condition that said interior bag (2) loses its mechanical strength during the sterilisation process to an extent that it tears open.

2. Dual bag according to Claim 1,
**characterised in**
that said exterior bag (1) presents breakthroughs, particularly holes (4) or slits.

3. Dual bag according to Claim 1 or 2,
**characterised in**
that said exterior bag (1) is transparent.

4. Dual bag according to any of the preceding Claims,
**characterised in**
that said interior bag (2) is coloured.

5. Dual bag according to any of the preceding Claims,
**characterised in**
that said interior bag (2) is heat-sealed, sewn or adhesively attached to said exterior bag (1) along a joining seam (5).

6. Dual bag according to any of the preceding Claims,
**characterised in**
that said exterior bag (1) has a configuration longer than that of said interior bag (2) and projects beyond the latter at the lower and upper edges.

7. Dual bag according to Claim 6,
**characterised in**
that said interior bag (2) is supported at a lower closure seam (3) of said exterior bag (1).

8. Dual bag according to any of the preceding Claims,
**characterised in**
that the material of said interior bag (2) softens or fuses at a temperature between 60 and 135 °C.

9. Dual bag according to Claim 8,
**characterised in**
that said interior bag (2) consists of a polyethylene which softens or fuses at temperatures as low as roughly 60 to 70 °C.

10. Dual bag according to any of the preceding Claims,
**characterised in**
that both said exterior bag (1) and said interior bag (2) consist of a chlorine-free synthetic resin.

## Revendications

1. Sac double stérilisable pour déchets infectieux ou matériaux similaires, comprenant une partie en résine synthétique, étanche aux liquides et essentiellement imperméable aux germes, et une partie perméable aux gaz, lesquelles parties sont reliées l'une à l'autre en particulier le long d'un joint de raccordement,
**caractérisé en ce**
que lesdites deux parties sont formées comme sacs séparés (1, 2) et combinées de façon que ladite partie étanche aux liquides est disposée en tant que sac intérieur (2) au-dedans du sac extérieur (1) perméable aux gaz, et a une constance thermique essentiellement plus basse que celle dudit sac extérieur (1) perméable aux gaz, étant entendu que ledit sac intérieur (2) perd sa résistance mécanique au cours de la stérilisation autant qu'il se déchire.

2. Sac double selon la revendication 1,
**caractérisé en ce**
que ledit sac extérieur (1) présente des percements à jour, en particulier des trous (4) ou fentes.

3. Sac double selon la revendication 1 ou 2,
**caractérisé en ce**
que ledit sac extérieur (1) est transparent.

4. Sac double selon une quelconque des revendications précédentes,
**caractérisé en ce**
que ledit sac intérieur (2) est coloré.

5. Sac double selon une quelconque des revendications précédentes,
**caractérisé en ce**
que ledit sac intérieur (2) est thermosoudé, cousu ou collé audit sac extérieur (1) le long d'un joint de raccordement (5).

6. Sac double selon une quelconque des revendications précédentes,
**caractérisé en ce**
que ledit sac extérieur (1) a une configuration plus longue que celle dudit sac intérieur (2) et se trouve en saillie du dernier au bord inférieur et au bord supérieur.

7. Sac double selon la revendication 6,
**caractérisé en ce**
que ledit sac intérieur (2) s'appuie à un joint de fermeture inférieur (3) dudit sac extérieur (1).

8. Sac double selon une quelconque des revendications précédentes,
**caractérisé en ce**
que la matière dudit sac intérieur (2) se ramollit ou se fond à une température entre 60 et 135 °C.

9. Sac double selon la revendication 8,
**caractérisé en ce**
que ledit sac intérieur (2) consiste en un polyéthylène qui se ramollit ou se fond déjà à une température à 60 - 70 °C environ.

10. Sac double selon une quelconque des revendications précédentes,
**caractérisé en ce**
que ledit sac extérieur (1) ainsi que ledit sac intérieur (2) consistent en une matière synthétique exempte de chlore.
